# EUROPEAN PATENT APPLICATION

(11) **EP 1 489 423 A1**
(43) Date of publication of application: **22.12.2004**
(21) Application number: 03291522.5
(22) Date of filing: 20.06.2003
(51) Int. Cl.: G01N 33/68, C12Q 1/68, C07K 14/47

(54) **Use of mitochondrial uncoupling proteins for diagnostic, prevention and treatment of diseases involving neuromuscular affection**

(71) Applicant: UNIVERSITE LOUIS PASTEUR, 67000 Strasbourg (FR)
(72) Inventor: Dupuis, Luc, 67200 Strasbourg (FR); Di Scala, Franck, 67100 Strasbourg (FR); De Tapia, Marc, 67114 Eschau (FR); Larmet, Yves, 67300 Schiltigheim (FR); Loeffler, Jean-Philippe, 67370 Berstett (FR)
(74) Representative: Becker, Philippe

(57) **Abstract**

The present invention relates generally to the fields of genetics and medicine. The present invention discloses the overexpression of UCP, more particularly UCP2 and UCP3, in diseases involving neuromuscular affection which can be used for the diagnosis, prevention and treatment of such diseases, and more particularly of amyotrophic lateral sclerosis, as well as for the screening of therapeutically active drugs.

SEQUENCE LISTING

<110> Université Louis Pasteur

<120> Use of mitochondrial uncoupling proteins for diagnostic, prevention
and treatment of diseases involving neuromuscular affection

<130> B0210EP

<160> 14

<170> PatentIn version 3.1

<210> 1
<211> 20
<212> DNA
<213> Artificial sequence

<220>
<223> Primer
<400> 1
gccttcagat ccaaggtgaa 20

<210> 2
<211> 20
<212> DNA
<213> Artificial sequence

<220>
<223> Primer
<400> 2
taagccggct gagatcttgt 20

<210> 3
<211> 20
<212> DNA
<213> Artificial sequence

<220>

<223>  Primer

<400>  3
ggggagagtc aagggctagt                                                    20

<210>  4

<211>  20

<212>  DNA

<213>  Artificial sequence

<220>

<223>  Primer

<400>  4
gctctgagcc cttggtgtag                                                    20

<210>  5

<211>  20

<212>  DNA

<213>  Artificial sequence

<220>

<223>  Primer

<400>  5
ggcccaacat cacaagaaat                                                    20

<210>  6

<211>  20

<212>  DNA

<213>  Artificial sequence

<220>

<223>  Primer

<400>  6
gcgttcatgt atcgggtctt                                                    20

<210>  7

<211>  20

<212>  DNA

<213>  Artificial sequence

<220>

<223>  Primer

<400>  7
cttgctaggt tgggagatgg                                                    20

<210>  8

<211>  20

<212>  DNA

<213>  Artificial sequence


<220>

<223>  Primer

<400>  8
tcacttttgc caaacacgac                                                    20

<210>  9

<211>  20

<212>  DNA

<213>  Artificial sequence


<220>

<223>  Primer

<400>  9
ttgatggtgc catatgatgc                                                    20

<210>  10

<211>  20

<212>  DNA

<213>  Artificial sequence


<220>

<223>  Primer

<400>  10
cggcagtgat tcatcagaaa                                                    20

<210>  11

<211>  20

<212>  DNA

<213>  Artificial sequence

<220>

<223>  Primer

<400>  11
accacagtcc atgccatcac                                                    20

<210>  12

<211>  20

<212>  DNA

<213>  Artificial sequence

<220>

<223>  Primer

<400>  12
tccaccaccc tgttgctgta                                                    20

<210>  13

<211>  20

<212>  DNA

<213>  Artificial sequence

<220>

<223>  Primer

<400>  13
atttcaggcc agcatacacc                                                    20

<210>  14

<211>  20

<212>  DNA

<213>  Artificial sequence

<220>

<223>  Primer

<400>  14
agtccagcag cttctccttg                                                    20

## Description

### Introduction

The present invention relates generally to the fields of genetics and medicine. The present invention discloses the overexpression of UCP, more particularly UCP2 and UCP3, in diseases involving neuromuscular affection which can be used for the diagnosis, prevention and treatment of such diseases, and more particularly of amyotrophic lateral sclerosis, as well as for the screening of therapeutically active drugs.

### Background

Amyotrophic lateral sclerosis is a fatal neurodegenerative disorder affecting the motor system. The death of spinal motor neurons leads to limb weakness with muscle atrophy and to a progressive involvement of respiratory muscles leading to death within 3 to 5 years after the onset. ALS is one of the most devastating disorders that affect the function of nerves and muscles. Based on U.S. population studies, about 5,600 people in U.S. are diagnosed with ALS each year. It is estimated that as many as 30,000 Americans have the disease at any given time.

Several mechanisms have been put forward to explain motor neuron degeneration, including oxidative stress, glutamate excitotoxicity and apoptosis, suggesting that the disease is multifactorial in origin. However, the precise molecular mechanisms responsible for the selective loss of motor neurons remain obscure. Five to 10 % of ALS cases are dominantly inherited, and genetic linkage studies revealed that 20 % of these are associated with point mutations in the gene encoding cytosolic Cu/Zn superoxide dismutase (SOD1), a major free radical scavenging enzyme that protects cells against oxidative stress. The identification of such mutations has allowed the production of transgenic mice that develop neurological disorders characteristic of ALS. It has been demonstrated that the deleterious mechanism of these mutations is related to a gain of function probably mediated by the mitochondrial fraction of the mutated SOD1.

A growing body of evidence involves mitochondrial dysfunction in ALS : morphological abnormamities of mitochondria (swelling) is a very early event in ALS and several reports demonstrated a decrease in mitochondria DNA as well as in respiratory chain enzyme activities both in ALS patients and in ALS transgenic mice. Furthermore, the involvement of mitochondria in ALS pathology is reinforced by the observation that supplementation of creatine, a mitochondrial energy buffering agent is the most efficient treatment known to delay motor dysfunction and extend survival of ALS mice. The mitochondrial respiratory chain is the major site of superoxide production and there is increasing evidence for the hypothesis of an oxidative stress-related mitochondrial involvement as a key determinant of motor neuron degeneration. Increased markers of lipid and protein damage due to oxidative stress have been detected in mitochondria of ALS mice. These findings support the concept that the mitochondrial fraction of SOD1 might gain its toxic effect via an aberrant catalytic function leading to excessive production of free radical species. In addition, markers of oxidative damage to protein, lipid and DNA have been detected in nervous system of both sporadic and familial ALS.

ALS is a very difficult disease to diagnose. No one test can provide a definitive diagnosis of ALS. The diagnosis of ALS is exclusively based on clinical examination, electromyographic studies and exclusion of other degenerative disorders. Physicians usually conduct a neurologic examination at regular intervals to assess whether symptoms such as muscle weakness, atrophy of muscles, hyperreflexia and spasticity are getting progressively worse. This diagnostic is long and costly making the lack of specific biological markers highly detrimental for an early beginning of appropriate and efficient therapies. Indeed, treatments are more efficient if they are administered at early stages of the disease. There is a genetic test based on the detection of the mutated SOD1, however this test concerns only about 20 % of the familial ALS. Hence, no diagnostic test of ALS is available. ALS is rather diagnosed by ruling out others diseases and disorders. Therefore, there is a strong need of diagnosing method of ALS. Earlier diagnosis is of key importance in the present but also in the future therapeuthic trials.

Until recently, there was no real treatment for ALS and therapy was aimed only at relieving pain, relaxing muscle cramps or easing other symptoms and complications. The FDA recently approved the first drug, riluzole, which may slow the degeneration for some people with ALS. Riluzole is believed to modulate the release of glutamate. Riluzole does not cure the disease or improve symptoms, but may extend the survival of patients by approximately three months. Riluzole appears to be more effective in patients with bulbar-onset than for those with limb-onset.

Celebrex is a new FDA-approved drug for the treatment of osteoarthritis, rheumatoid arthritis and pain. The active ingredient Celecoxib is a nonsteroidal anti-inflammatory drug (NSAIDS) that specifically targets COX-2. Johns Hopkins University has tested Celebrex in the SOD mouse model and has shown that it extends the life of the mouse by approximately 25%. In addition in rat spinal cord slices that are a model used to measure protection of motor neurons, Celebrex significantly protected neurons against chronic injury by glutamate. There is also significant epidemiology data supporting the neuroprotective effects of anti-inflammatory drugs in Alzheimer's and several studies are now ongoing evaluating their effectiveness in that model.

Co-enzyme Q10 is a non-prescription dietary supplement, or nutraceutical. CoQ10 is an endogenously synthesized provitamin that is involved in a variety of cellular processes. It plays a vital role in ATP production. Also, it has been noted to have antioxidant properties, such as free-radical scavenging. CoQ10 is indicated for heart failure and mitochondrial cytopathies. In a study, done at Massachusetts General Hospital in Boston, they investigated whether it can exert neuroprotective effects in a variety of animal models. They found that CoQ10 did extend the survival in a transgenic mouse model of ALS. They also demonstrated positive findings in Parkinson's and Huntington's Disease, therefore the studies raise prospect that administration of CoQ10 may be useful for the treatment of neurodegenerative diseases.

Creatine is a non-prescription dietary supplement that is promoted for its ability to enhance muscle strength and physical endurance. This dietary supplement is similar to the natural compound creatine phosphate, which is an essential component of the energy-building system in muscle cells. The significant correlation between creatine and ALS was cited in a study of SOD mice, published in Nature Medicine in 1999. The studies findings showed with oral administration of creatine in the diet, the mice had significant improvements in their survival. With supplemented diets, the SOD mice life extension was 9-18%. Therefore, creatine is a supplement that could be beneficial to ALS patients

Therapies based on stem cells are the subject of intense research in ALS. However, the efficiency of these therapies in ALS has not yet been proved.

Hence, the existing treatments are able to slightly delay the degeneration. Therefore, there is a strong need of efficient treatment that cure ALS or, at least, notably extend the survival of patients.

### Summary of the invention

The present invention provides various methods for diagnosing, preventing and treating various diseases involving neuromuscular affection, particularly amyotrophic lateral sclerosis (ALS). Methods for screening agents to identify agents useful in treating diseases involving neuromuscular affection are also provided.

More particularly, the invention concerns a method of diagnosing the occurrence of a disease involving neuromuscular affection or assessing a patient's susceptibility to a disease involving neuromuscular affection by detecting in a patient sample an elevated level of UCP2 or UCP3 expression. In some methods, detection is accomplished by detecting elevated levels of UCP2 and/or UCP3 transcript. Other methods involve detecting an elevated level of UCP2 and/or UCP3 polypeptide. Optionally, the method comprises the detection of the elevated level of UCP2 expression. Optionally, the method comprises the detection of the elevated level of UCP3 expression. Optionally, the method comprises the detection of the elevated level of UCP2 and UCP3 expression. Preferably, the detection of the elevated level of UCP2 and/or UCP3 expression is carried out in skeletal muscle sample. Preferably, the disease involving neuromuscular affection is amyotrophic lateral sclerosis (ALS). Optionally, the disease involving neuromuscular affection is a diabetic neuropathy or a myopathy.

The invention also concerns a method of assessing the evolution of a disease involving neuromuscular affection in a patient by determining the level of UCP2 and/or UCP3 expression in a patient sample. In some methods, the level of UCP2 and/or UCP3 expression is determined by the level of UCP2 and/or UCP3 transcript. Other methods involve the determination of the level of UCP2 and/or UCP3 polypeptide. Preferably, the determination of the level of UCP2 and/or UCP3 expression is carried out in skeletal muscle sample. Preferably, the disease involving neuromuscular affection is amyotrophic lateral sclerosis (ALS). Optionally, the disease involving neuromuscular affection is a diabetic neuropathy or a myopathy. Preferably, the method comprises an additional step of comparing the expression level to a reference expression level in a sample from said subject at an earlier time point.

The invention further concerns a method of determining the efficacy of a treatment of a disease involving neuromuscular affection in a patient by determining the level of UCP2 and/or UCP3 expression in a patient sample. In some methods, the level of UCP2 and/or UCP3 expression is determined by the level of UCP2 and/or UCP3 transcript. Other methods involve the determination of the level of UCP2 and/or UCP3 polypeptide. Preferably, the determination of the level of UCP2 or UCP3 expression is carried out in skeletal muscle sample. Preferably, the disease involving neuromuscular affection is amyotrophic lateral sclerosis (ALS). Optionally, the disease involving neuromuscular affection is a diabetic neuropathy or a myopathy. Preferably, the method comprises an additional step of comparing the expression level to a reference expression level in a sample from said subject prior to or at an earlier stage of the treatment. A decreased level of UCP2 and/or UCP3 is indicative of the efficacity of the treatment.

The invention concerns a method of selecting biologically active compounds against a disease involving neuromuscular affection comprising the steps of : (a) administering a test compound to a subject; (b) determining the level of UCP2 and/or UCP3 expression in a subject sample; (c) comparing the expression level of UCP2 and/or UCP3 to a reference expression level of UCP2 and/or UCP3 in a sample from said subject prior to the administration of the test compound or at an earlier stage after the test compound administration. Preferably, the determination of the level of UCP2 or UCP3 expression is carried out in skeletal muscle sample. Preferably, said subject is a non-human mammal such as a mouse, a rat, etc...

The invention concerns a method of screening and selecting a compound inhibiting the activity of UCP2 and/or UCP3, which are useful for a treatment against a disease involving neuromuscular affection, comprising the steps of : (a) contacting a test compound with a cell expressing UCP2 and/or UCP3; (b) determining the expression level and/or activity of UCP2 and/or UCP3. Optionally, the method comprises a step of comparing the expression level of UCP2 and/or UCP3 to a reference expression level of UCP2 and/or UCP3 in a cell expressing UCP2 and/or UCP3 without any test compound.

The invention also concerns a method of treatment of a disease involving neuromuscular affection comprising administering a therapeutic amount of a compound inhibiting the activity of UCP2 and/or UCP3. Said compound can be selected by a method according to the present invention. Optionally, said compound inhibits the expression of UCP2 and/or UCP3. Preferably, said compound is an antisense or an RNAi against UCP2 and/or UCP3 transcript. Optionally, said compound is an antibody directed against UCP2 and/or UCP3. Preferably, said antibody inhibits the activity of UCP2 and/or UCP3.

The invention concerns a pharmaceutical composition comprising a compound inhibiting the activity of UCP2 and/or UCP3. Said compound can be selected by a method according to the present invention. Optionally, said compound inhibits the expression of UCP2 and/or UCP3. Preferably, said compound is an antisense or an RNAi against UCP2 and/or UCP3 transcript. Optionally, said compound is an antibody directed against UCP2 and/or UCP3. Preferably, said antibody inhibits the activity of UCP2 and/or UCP3.

The invention also concerns the use of a compound inhibiting the activity of UCP2 and/or UCP3 for the preparation of a drug for treating a disease involving neuromuscular affection. Said compound can be selected by a method according to the present invention. Optionally, said compound inhibits the expression of UCP2 and/or UCP3. Preferably, said compound is an antisense or an RNAi against UCP2 and/or UCP3 transcript. Optionally, said compound is an antibody directed against UCP2 and/or UCP3. Preferably, said antibody inhibits the activity of UCP2 and/or UCP3. Preferably, the disease involving neuromuscular affection is amyotrophic lateral sclerosis (ALS). Optionally, the disease involving neuromuscular affection is a diabetic neuropathy or a myopathy.

### Legend to Figures

**Figure 1** : Phylogenetic analysis and tissue distribution of UCPs in mice
   Figure 1A: phylogenetic tree of murine mitochondrial carrier proteins. UCP gene family is indicated by the dashed line.
   Figure 1B: UCP family expression in mouse tissues. Semi-quantitative RT-PCR were performed in five tissues (BAT : Brown adipose tissue ; WAT : white adipose tissue ; liver ; SkM : skeletal muscle ; LSC : lumbar spinal cord) for each of the five members of murine UCPs. 26 or 30 PCR cycles were performed to ensure the linearity of amplification.
**Figure 2** : UCP2 and UCP3 expression in the gastrocnemius muscle of ALS transgenic mice.
   Figure 2A: Representative RT-PCR showing UCP2 and UCP3 mRNA levels in the gastrocnemius muscle of wild-type (+) and G86R (M) mice of 75 (75d), 90 (90d), and 105 days (105d) old. G3PDH mRNA levels were used as internal control. Controls without RT (not shown) and with different dilutions of cDNA template (not shown) were performed to ascertain the specificity and linearity of the amplification.
   Figure 2B: Quantitative analysis of RT-PCR. The level of mRNA expression in wild-type mice is 1. The data represent means ± SE (n=7). * p<0,05 (*vs* wt) ***P*<0.01 (*vs* wt), one-way ANOVA followed by Newman-Keuls multiple comparison test.
**Figure 3 :** UCP2 and UCP3 expression in denervation models and wild-type SOD1 transgenic mice
   Figure 3A: Fingerprint traces of sham-operated and sciatic nerve-crushed mice. Note the different trace of the ipsilateral hindleg in crushed animals as compared to the contralateral hindleg.
   Figure 3B: Histological examination of toluidin blue-stained sections of sciatic nerve in healthy (sham-operated and wild-type (Wt) mice), crushed and G86R mice. Note the axonal degeneration in the latter two groups.
   Figure 3C: Representative RT-PCR showing UCP2, UCP3 and AChRα mRNA levels in the gastrocnemius muscle of sciatic nerve-crushed mice. Controls as in Figure 2.
   Figure 3D: Quantitative analysis of RT-PCR performed in crushed mice. The data represent means ± SE (n=5). **P*<0.05, ***P*<0.01 (*vs* contralateral muscle); one-way ANOVA followed by Newman-Keuls multiple comparison test.
   Figure 3E: Representative RT-PCR showing UCP2 and UCP3 mRNA levels in the gastrocnemius muscle of 105 days old wild-type SOD1 mice (Tg) and the corresponding non-transgenic littermates (+).
   Figure 3F : Quantitative analysis of Panel E experiments (n=3)
**Figure 4**:UCP3 expression in ALS patients.
   Figure 4A: Representative RT-PCR showing UCP3 mRNA levels in muscle biopsies of ALS (ALS), polyneuropathies (PN) or primary muscle diseases (PMD) patients
   Figure 4B: Quantitative analysis of RT-PCR experiments. UCP3 mRNA levels are expressed relative to 18S rRNA levels as determined by denaturing agarose electrophoresis (not shown). *p<0,05 Mann-Whitney test.
   Figure 4C: Representative Western blot showing UCP3 protein extracted from muscle biopsies of ALS patients and normal subjects (Ct)

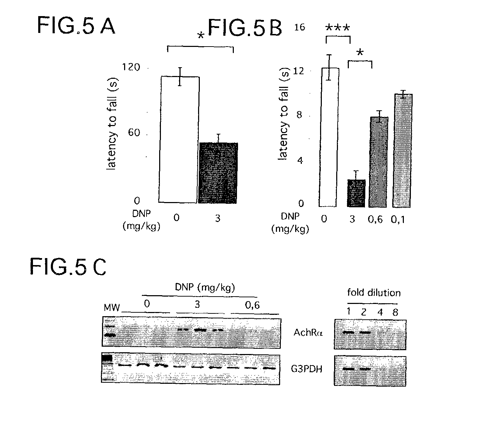

**Figure 5**: Analysis of DNP-injected mice
   Figure 5A: Accelerating rotarod test. Mice were injected either with 3 mg/kg 2,4-dinitrophenol (DNP) (3) or with vehicle (0). N=5 per group; *p<0,05 Mann-Whitney test
   Figure 5B: Grip test. Mice were injected either with 3 mg/kg 2,4-dinitrophenol (DNP) (3), 0,6 (0,6), 0,1 (0,1) or with vehicle (0). N=5 per group; ***p<0,001; *p<0,05 Mann-Whitney test
   Figure 5C: Representative RT-PCR showing AchRα mRNA levels. Abbreviations as in panel B.

### Detailed Description of the Invention

Uncoupling proteins are members of the growing family of mitochondrial carrier proteins. The founding member of UCP family is UCP1, formerly known as thermogenin. UCP1 is almost exclusively expressed in the brown adipose tissue and is responsible, through the diversion of energy from ATP synthesis to thermogenesis, for the thermogenic function of this tissue, especially in rodents. UCP 2, 3, 4, and 5 (also known as BMCP1) were further cloned by homology with UCP1 and their thermogenic activity *in vivo* is still controversial. Mitochondria from UCP3 knock-out mice display a higher conductance rate although no changes are noted in the overall respiratory rate of the animal. Such data suggest that UCPs might have function in the fine regulation of mitochondrial respiration and it was recently suggested that this function deals with the resistance to oxidative stress. Furthermore, it was very recently demonstrated that UCP2 controlled a particular form of cell death called oncosis. This shows that UCPs are linked to oxidative stress resistance, mitochondrial function and ultimately cell survival, these three functions being defective in ALS.

The inventors decided to monitors UCPs' expression pattern in ALS. The inventors showed that UCPs, and particularly UCP2 and UCP3, were upregulated in skeletal muscle but not in spinal cord. Consistent with this pattern of expression, ATP levels were selectively depleted in muscle but not in neural tissues. UCP3 upregulation was not observed in experimentally denervated muscles, suggesting that changes in muscular UCP3 expression are associated with the physiopathological processes of ALS. This is further supported by the inventors' observation of increased UCP3 levels in human ALS muscular biopsies. The inventors also observed that UCP2 overexpression could be linked to denervetion processes. In addition, the inventors showed that chronic mitochondrial *uncoupling in vivo* is sufficient to trigger a syndrome with some ALS features. UCP3 upregulation in skeletal muscle may thus contribute to the characteristic mitochondrial damage of ALS and to onset of the disease. Moreover, since skeletal muscle is a key metabolic tissue, the inventors findings suggest that ALS may not solely arise from neuronal events but also from more generalized metabolic defects.

Gonzalez-Zulueta et al (WO 02/36829) described that UCP2 inhibits certain components of an apoptotic cascade. They observed an increase in UCP2 expression in response to a neurological insult. Therefore, they deduced that UCP2 may have a protective effect and suggested a treatment method of a neurological disorder by administering an agent that increases the activity of UCP2.

The modulation of UCP2 and UCP3 has been described for the treatment of obesity and cachexia (WO 99/00123; WO 98/50542 ; WO 98/45313), or for the treatment of heart failure (WO 01/96398).

The terms "UCP2" and "UCP3" respectively refer to UCP2 and UCP3 nucleic acid or polypeptide. UCP 2 or 3 polypeptide or protein refers to a protein having a native UCP 2 or 3 amino acid sequence, as well as variants and modified forms thereof. For example, an amino acid sequence of UCP2 is described in Accession number P55851 and an amino acid sequence of UCP2 is described in Accession number NP073714. UCP 2 or 3 nucleic acid includes nucleic acids that encode for the various UCP proteins.

A "disease involving neuromuscular affection" can be, but are not limited to, a neurodegenerative disease, a neuromuscular disease, or a motor neuron disease. Preferably, a metabolic neuronal disease is selected from the group consisting of Alzheimer's disease, Parkinson's disease, Huntington's disease, amyotrophic lateral sclerosis, myopathy, dementia, diabetic neuropathies, spinal muscular atrophies (SMA), primary lateral sclerosis (PLS), inherited ataxia, mitochondrial encephalopathy, progressive muscular atrophy, postpolio syndrome, diabetic neuropathy and muscular dystrophy. Preferably, said disease is an amyotrophic lateral sclerosis, a myopathy, or a diabetic neuropathy. More preferably, said disease is an amyotrophic lateral sclerosis (ALS).

### DIAGNOSIS AND MONITORING

The invention now provides diagnosis methods of a disease involving neuromuscular affection based on a monitoring of an elevated level of UCP2 or UCP3 expression in a subject. These methods allow an early diagnosis of a disease involving neuromuscular affection. Within the context of the present invention, the term "diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmaco-genetics), etc.

More specifically, certain methods involve diagnosing the occurrence of a disease involving neuromuscular affection or assessing a patient's susceptibility to a disease involving neuromuscular affection by detecting in a patient sample an elevated level of UCP2 and/or UCP3 expression. The presence of said elevated level of expression is indicative of the occurence of or the susceptibility to a disease involving neuromuscular affection. Preferably, the elevated level is determined by comparison with a reference level determined with healthy population. Optionally, the method comprises the determination of the level of UCP2 expression. Optionally, the method comprises the determination of the level of UCP3 expression. Optionally, the method comprises the determination of the level of UCP2 and UCP3 expression. This method allows an early diagnosis of a disease involving neuromuscular affection, more particularly through the UCP3 expression level.

Some of the diagnostic methods provided herein involve assessing the evolution of a disease involving neuromuscular affection in a patient by determining the level of UCP2 and/or UCP3 expression in a patient sample. The presence of said decreased level of expression is indicative of a health gain for the patient. The presence of said elevated level of expression is indicative of a health worsening for the patient. Preferably, the level of UCP2 and/or UCP3 expression in a patient sample is compared to the level of UCP2 and/or UCP3 expression in a patient sample at an earlier time point. Optionally, the method comprises the determination of the level of UCP2 expression. Optionally, the method comprises the determination of the level of UCP3 expression. Optionally, the method comprises the determination of the level of UCP2 and UCP3 expression.

The invention further concerns a method of determining the efficacy of a treatment of a disease involving neuromuscular affection in a patient by determining the level of UCP2 and/or UCP3 expression in a patient sample. The presence of said decreased level of expression is indicative of the efficacy of the treatment. The presence of said elevated level of expression is indicative of the inefficiency of the treatment. Preferably, the level of UCP2 and/or UCP3 expression in a patient sample is compared to the level of UCP2 and/or UCP3 expression in a sample from said subject prior to or at an earlier stage of the treatment. Optionally, the method comprises the determination of the level of UCP2 expression. Optionally, the method comprises the determination of the level of UCP3 expression. Optionally, the method comprises the determination of the level of UCP2 and UCP3 expression.

In a particular embodiment of the above-mentionned methods, the methods comprise the determination of the level of UCP2 expression. In an alternative embodiment, the methods comprise the determination of the level of UCP3 expression. In a further embodiment, the methods comprise the determination of the level of UCP2 and UCP3 expression.

Preferably, the assays used for the determination of the expression levels of UCP2 or UCP3 are respectively specific of UCP2 or UCP3. More particularly, these assays are able to discriminate between UCP2 and UCP3.

In some methods, detection is accomplished by determining levels of UCP2 and/or UCP3 transcript. Other methods involve determining the level of UCP2 and/or UCP3 polypeptide.

Preferably, said disease involving neuromuscular affection is amyotrophic lateral sclerosis (ALS).

By "elevated level" is intended that the level of expression in the patient sample is higher than the level expression in a reference sample. For example, the expression level in a reference sample can be the expression level for a sample obtained from the same individual at a different time point. Alternatively, the expression level for a reference sample is a value determined for a control cell or individual, or a statistical value (e. g., an average or mean) established for a population of control cells or individuals. Thus, for instance, a control individual or control population can include healthy individuals, more particularly those not suffered from a disease involving neuromuscular affection or those not susceptible to a metabolic neuronal disease. The population that serves as a control can vary in size, having as few as a single member, but potentially including 10, 100, 1,000, 10,000 or more individuals.

Samples can be obtained from a variety of sources. Preferably, said samples are obtained from a human subject. However, some samples can be obtained from others mammals such as primates, mice and rats. Samples can be obtained from tissues or fluids of an individual, as well as from cell cultures or tissue homogenates. Samples can be derived from *in vitro* cell cultures. Preferably, the patient's sample is provided by biopsy, more particularly, a muscular biopsy. Preferably, the detection of the elevated level of UCP2 or UCP3 expression is carried out in a sample from muscle, more particularly skeletal muscle. The muscular biopsies are easily accessible and can be processed under local anaesthesia with low traumatic consequences for patients.

When the method involves the determination of transcript level, the method can comprise the lysis of the cells contained in the sample and the purification of nucleic acids. The level of UCP2 and/or UCP3 expression can be quantitated by a number of established techniques, including, but not limited to, Northern-Blot, RNase protection assays (RPA), nucleic acid probe arrays, quantitative PCR, dot blot assays and in situ hybridization. The level of expression can measured by mRNA, cDNA, cRNA or any nucleic acid product derived thereof, for example by amplification, transcription or reverse-transcription.

The level of UCP2 and/or UCP3 polypeptide can be determined by the quantification of UCP2 and/or UCP3 protein or by the measure of UCP2 and/or UCP3 activity. This can be accomplished by a number of different approaches among which the use of antibodies specific of UCP2 and/or UCP3 protein (e.g., Western blot, ELISA, radio-immunoassays (RIA) and immuno-enzymatic assays (IEMA)), and assays measuring the UCP2 and/or UCP3 activity. The UCP2 and/or UCP3 activity can be detected through the mitochondrial respiration mediated by UCP2 and/or UCP3. Assays can be conducted using isolated cells or tissue samples, or isolated mitochondrial preparations. Alternatively, the UCP2 and/or UCP3 activity can be detected by measuring the extent of mitochondrial swelling. Methods for conducting such mitochondrial assays are known in the art (e.g., Salvioli et al, 1997; Smiley et al, 1997; WO 00/17353; WO 98/45313).

The invention also relates to a diagnostic kit for diagnosing the occurrence of a disease involving a neuromuscular affection or assessing a patient's susceptibility to a disease involving a neuromuscular affection, for assessing the evolution of a disease involving a neuromuscular affection in a patient, or for determining the efficacy of a treatment of a disease involving a neuromuscular affection in a patient, comprising products and reagents for determining in a sample from a subject the level of UCP2 and/or UCP3 expression. Said diagnostic kit according to the present invention comprises at least one element selected from the group consisting of any primer or any pair of primers suitable for amplifying UCP2 and/or UCP3, any nucleic acid probe specific for UCP2 and/or UCP3 and/or any antibody specific of UCP2 and/or UCP3. Said kit can also comprises means to determine the reference expression level of UCP2 and/or UCP3. Said diagnostic kit according to the present invention can comprise reagents and/or protocols for performing a hybridization, amplification or antigen-antibody immune reaction.

### SCREENING

The screening methods generally involve conducting various types of assays to identify compounds that downregulate the expression or activity of UCP2 and/or UCP3. Such screening methods can initially involve to identify compounds that can bind UCP2 and/or UCP3 polynucleotides or polypeptides. Certain assays are designed to measure more clearly the effect that different compounds have on UCP2 and/or UCP3 activities and/or expression levels.

A variety of screening methods is provided. Certain of these methods involve screening for identifying a compound useful for treating a disease involving a neuromuscular affection (e. g., ALS) by identifying a compound that downregulates UCP2 and/or UCP3 expression and/or inhibits UCP2 and/or UCP3 activity in a subject. Some of the screening methods involve: (a) administering to a subject a test compound, wherein the subject is a mammal other than a human; and (b) determining in a sample from the subject the expression level of UCP2 and/or UCP3 to identify the compound that downregulates UCP2 and/or UCP3 expression in the subject. Others screening methods comprise (a) administering to a subject a test compound, wherein the subject is a mammal other than a human; and (b) determining in a sample from the subject the activity of UCP2 and/or UCP3 to identify the compound that inhibits UCP2 and/or UCP3 activity in the subject.

The invention concerns a method of selecting biologically active compounds against a metabolic neuronal disease comprising the steps of : (a) administering a test compound to a subject; (b) determining the level of UCP2 and/or UCP3 expression in a subject sample; (c) comparing the expression level of UCP2 and/or UCP3 to a reference expression level of UCP2 and/or UCP3 in a sample from said subject prior to the administration of the test compound or at an earlier stage after the test compound administration.

Preferably, said subject is an animal model of a disease involving a neuromuscular affection (e.g., SOD1 G86R, G85R, G37R transgenic mice). Optionally, said subject reproduces the symptom of the disease.

The invention concerns a method of screening a compound inhibiting the activity of UCP2 and/or UCP3 comprising the steps of : (a) contacting a test compound with a cell expressing UCP2 and/or UCP3; (b) determining the expression level and/or activity of UCP2 and/or UCP3.

The cell expressing UCP2 and/or UCP3 can be, but is not limited to, a cell naturally expressing at least one of these proteins (e.g., for UCP2 : liver, white adipose tissues, spinal cord ; for UCP3 : skeletal muscle, white adipose tissues, brown adipose tissues, spinal cord), or a cell transformed with an expression vector for UCP2 and/or UCP3. Optionally, said cell can be a neuronal cell or a skeletal muscle cell.

Compounds identified during these screening can serve as the basis for the development of more active analogs. These compounds and/or the active analogs generated therefrom can be used in a method of treatment of a metabolic neuronal disease, formulated into pharmaceutical compositions effective in treating a disease involving a neuromuscular affection and used for the preparation of a drug for treating a disease involving a neuromuscular affection.

### TREATMENT

The invention also concerns a method of treatment of a disease involving a neuromuscular affection comprising administering a therapeutic amount of a compound decreasing the activity of UCP2 and/or UCP3.

By "treatment" is intended both therapeutic treatment and prophylactic or preventative measures. The object of the treatment is to prevent or slow down the progression of a disease involving a neuromuscular affection. Those in need of treatment include those already with the disease as well as those prone to have the disease or those in which the disease is to be prevented.

The compound that decreases the activity of UCP2 and/or UCP3 can be a compound that inhibits the expression or synthesis of UCP2 and/or UCP3, a compound that inhibits the activity of UCP2 and/or UCP3.

Compounds of interest are any molecule that inhibits UCP2 and/or UCP3 expression or activity. Preferably, the compounds are selected from the group consisting of a purine nucleotide (e.g. a T3 inhibitor such as propyl-thio uracile), antisense or RNAi specific to UCP2 and/or UCP3, inhibitors of transcription factors specific to UCP2 and/or UCP3, inhibitors of a hormone inducing UCP2 and/or UCP3 expression, antibodies directed against UCP2 and/or UCP3 or against a molecule interacting with one of them which are able to decrease UCP2 and/or UCP3 activity. More preferably, said compound is an antisense or a RNAi specific to UCP2 and/or UCP3.

Said compound can be selected by a method according to the present invention.

Optionally, said compound inhibits the expression level of UCP2 and/or UCP3. Preferably, said compound is an antisense or an RNAi against UCP2 and/or UCP3 transcript. Optionally, said compound is an antibody directed against UCP2 and/or UCP3.

The invention concerns a pharmaceutical composition comprising a compound inhibiting the activity of UCP2 and/or UCP3. Said compound can be selected by a method according to the present invention. Optionally, said compound inhibits the expression level of UCP2 and/or UCP3. Preferably, said compound is an antisense or an RNAi against UCP2 and/or UCP3 transcript. Optionally, said compound is an antibody directed against UCP2 and/or UCP3.

As used herein, the term "pharmaceutical composition" refers to a composition comprising a compound inhibiting the activity of UCP2 and/or UCP3 together with a pharmaceutically acceptable carrier or diluent. A pharmaceutical composition of the present invention is directed to a composition suitable for the treatment of a metabolic neuronal disease. A pharmaceutically acceptable carrier includes, but is not limited to, physiological saline, ringers, phosphate buffered saline, and other carriers known in the art. Pharmaceutical compositions may also include stabilizers, anti-oxidants, colorants, and diluents.

Pharmaceutically acceptable carriers and additives are chosen such that side effects from the pharmaceutical compound are minimized and the performance of the compound is not canceled or inhibited to such an extent that treatment is ineffective. Thus, the compound inhibiting the activity of UCP2 and/or UCP3 can be administered individually or together in any conventional oral, parenteral, rectal or transdermal dosage form.

The present invention provides composition comprising a compound inhibiting the activity of UCP2 and/or UCP3 that is effective to a disease involving a neuromuscular affection and routes for administering the composition to the patient. An amount of the compound that is effective for treating a metabolic neuronal disease is administered to the patient. Typically, an effective dosage of an agent for a 50 kg to 100 kg human being is in the range of about 0.1 mg to 3000 mg per day in single or divided doses, and preferably about one mg to about 1000 mg per day in single or divided doses. More preferably, a dosage range is 10 mg to 100 mg per day in single or divided doses.

The amount and timing of the compound administration is dependent upon the subject being treated, on the severity of the affliction, on the manner of administration and upon the judgment of the prescribing physician.

The invention also concerns the use of a compound inhibiting the activity of UCP2 and/or UCP3 for the preparation of a drug for treating a disease involving a neuromuscular affection. Said compound can be selected by a method according to the present invention. Optionally, said compound inhibits the expression level of UCP2 and/or UCP3. Preferably, said compound is an antisense or an RNAi against UCP2 and/or UCP3 transcript. Optionally, said compound is an antibody directed against UCP2 and/or UCP3.

Certain treatment methods provided herein involve treating a subject having or being susceptible to a disease involving a neuromuscular affection by administering to the subject an effective amount of an agent that decreases the activity of UCP2 and/or UCP3. The disease involving a neuromuscular affection that are amenable to the methods include, but are not limited to, amyotrophic lateral sclerosis, diabetic neuropathy, and myopathy. If the subject is susceptible to the disease involving a neuromuscular affection, the subject is administered a prophylactic amount of the agent prior to occurring of the disease. If, however, the subject has already suffered the disease involving a neuromuscular affection, then the subject is administered a therapeutic amount of the agent. The agent which decreases the activity of UCP2 and/or UCP3 can be co-administered with various other agents, including, for example, agents that increase permeability of the blood/brain barrier and/or blood anticoagulants.

Certain treatment methods involve administering agents that inhibit the synthesis or expression of UCP2 and/or UCP3 or a UCP2 and/or UCP3 inhibiting agent. In some methods, the agent administered is a nucleic acid that encodes an antisense of UCP2 and/or UCP3 or a UCP2 and/or UCP3 inhibitor. In such instances, the nucleic acid can be inserted into a viral vector or other expression vectors. The viral vector can also include a promoter operably linked to the nucleic acid which selectively drives expression in nerve or muscular cells. The promoter can be a UCP2 and/or promoter or a heterologous promoter. In certain methods, the viral vector is introduced into the cerebrospinal fluid; in other methods, the vector is injected into the intraventricular space or into muscle. Still other treatment methods also involve producing ex vivo genetically-modified neuronal or muscular stem cells that harbor the vector that includes a nucleic acid encoding an antisense of UCP2 and/or UCP3. The modified stem cells are then introduced into the intracerebroventricular space, into the cerebrospinal fluid or into muscle.

### Examples

### MATERIALS AND METHODS

### Animals and tissue preparation

The inventors used transgenic male mice with the missense mutation Gly86Arg (G86R, human G85R equivalent) in the SOD1 gene (Ripps et al, 1995) of 75, 90 and 105 days old. Non-transgenic littermates served as wild-type controls and for denervation and DNP injection experiments. All mice were from the FVB/N strain. Peripheral nerve injury in normal male mice was accomplished by either crush or axotomy of sciatic nerve as previously described (Dupuis et al, 2002a and 2002b). Sham-operated mice served as control. The inventors assessed motor dysfunction by monitoring fingerprint traces after dipping hindleg extremities in ink. Lumbar spinal cords and gastrocnemius muscles were dissected either from transgenic mice or sciatic nerve-injured mice. Tissues were immediately frozen in liquid nitrogen and stored at -80°C until use. Animal manipulation followed current European Union regulations.

### Human tissue preparation

The inventors obtained muscle biopsies from patients submitted to either a standard surgical diagnosis procedure under local anaesthesia or an orthopaedic operation under general anaesthesia. All patients gave written informed consent. Tissues were immediately frozen in liquid nitrogen and stored at -80 °C until use. The ALS group consisted of 21 patients (7 for RT-PCR studies, 14 for western blot studies) with probable or definite sporadic ALS according to the World Federation of Neurology criteria (Brooks, 1994) and attending the ALS Center of the Pitié Salpétrière Hospital (Paris, France). Control groups included: 10 patients without any significant neurological history who underwent an orthopaedic surgery (western blot studies), 6 patients with symptomatic sensorimotor polyneuropathies and 3 patients with primary muscle diseases (RT-PCR studies).

### Phylogenetic analysis of UCP family

Proteins homologous to UCP1 were defined using a PSI-BLAST algorithm (NCBI, Bethesda). The 15 most highly homologous proteins were further selected and their sequences were aligned using CLUSTALW algorithm (Infobiogen, France). The results were further analysed using PHYLIP software and Fitsch-Margolias algorithm. Tree was rooted using the hemoglobin alpha chain as root.

### RT-PCR

Two µg of total RNA was reverse transcribed using 200 U MoMuLV reverse transcriptase and 0.5 µg random primers (Promega, Charbonnieres, France) as described (Sambrook et al, 1989). After PCR amplification, products were separated with a 2% agarose gel, and visualized by ethidium bromide staining. The degree of denervation in transgenic and sciatic nerve-injured mice was assessed by monitoring the levels of nicotinic acetylcholine receptor α subunit (AchRα) mRNA (Duclert et al, 1990). The inventors used either glyceraldehyde 3-phosphate dehydrogenase (G3PDH) mRNA levels or 18S rRNA levels as internal control. PCR primers were as follows:

Murine UCP1 5' primer: 5'- GCCTTCAGATCCAAGGTGAA -3' (SEQ ID No 1)

Murine UCP1 3' primer: 5'- TAAGCCGGCTGAGATCTTGT -3' (SEQ ID No 2)

Murine UCP2 5' primer: 5'- GGGGAGAGTCAAGGGCTAGT -3' (SEQ ID No 3)

Murine UCP2 3' primer: 5'- GCTCTGAGCCCTTGGTGTAG -3' (SEQ ID No 4)

Murine UCP3 5' primer: 5'- GGCCCAACATCACAAGAAAT -3' (SEQ ID No 5)

Murine UCP3 3' primer: 5'- GCGTTCATGTATCGGGTCTT -3' (SEQ ID No 6)

Murine UCP4 5' primer 5'-CTTGCTAGGTTGGGAGATGG-3' (SEQ ID No 7)

Murine UCP4 3' primer 5'-TCACTTTTGCCAAACACGAC-3' (SEQ ID No 8)

Murine BMCP1 5' primer: 5'- TTGATGGTGCCATATGATGC -3' (SEQ ID No 9)

Murine BMCP1 3' primer: 5'- CGGCAGTGATTCATCAGAAA -3' (SEQ ID No 10)

### Human and murine G3PDH

5' primer: 5'-ACCACAGTCCATGCCATCAC-3' (SEQ ID No 11)

3' primer: 5'-TCCACCACCCTGTTGCTGTA-3' (SEQ ID No 12)

### Human UCP3:

5' primer: 5'- ATTTCAGGCCAGCATACACC-3' (SEQ ID No 13)

3’ primer: 5’- AGTCCAGCAGCTTCTCCTTG -3’ (SEQ ID No 14)

### Anti-UCP3 Western blot

Tissues were homogenized in PBS containing 1% Nonidet P40, 0.5% sodium deoxycholate, 0.1 % SDS, 0.5% aprotinine, 5 mM DTT and 1 mM PMSF. Homogenates were then boiled for 5 min and sonicated for 30 seconds. After centrifugation, equal amounts of protein, according to Bradford protein assay (BioRad), were electrophoresed through a 13% SDS-polyacrylamide gel. Separated proteins were then electrotransferred to nitrocellulose membranes, and immunostained with the 3046 anti-UCP3 antibody (chemicon, CA, USA) diluted 1/1000. Membranes were then incubated with horseradish peroxidase-conjugated goat anti-rabbit IgG (Pierce, Bezons, France) diluted 1/2000, and developed by enhanced chemiluminescence detection. To ensure equal loading, membranes were stained with Ponceau S stain (Sigma).

### ATP levels measurements

ATP levels were measured using ATP bioluminescence assay kit CLS II (Roche) and a FB12 Berthold luminometer following provider's recommendations. Data were standardized versus the total protein content using a BCA kit (Roche).

### RESULTS

### Uncoupling proteins family pattern of expression in tissues involved in ALS

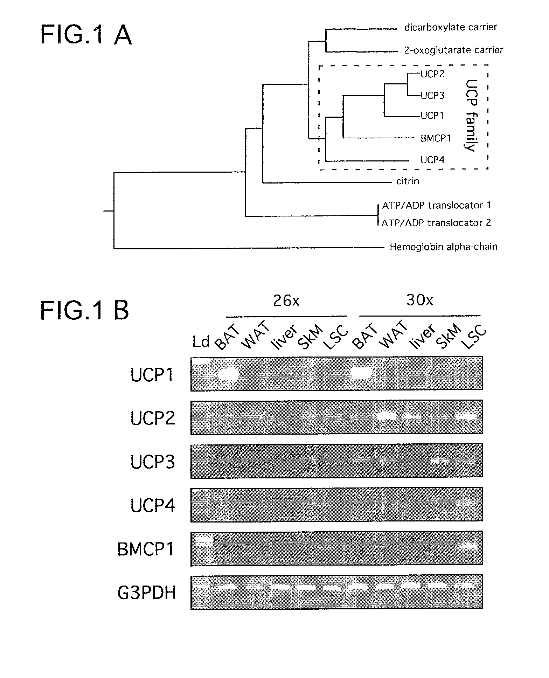

To date, murine UCP 1 to 4 have been cloned. The inventors identified murine BMCP1 sequence as RIKEN cDNA NM028711 by homology with human BMCP1 MRNA. To further clarify the evolutionnary relationships between UCPs and mitochondrial carrier proteins, the inventors built a phylogenic tree of murine mitochondrial carrier proteins (figure 1A). UCP1, 2 and 3 are very close relatives. BMCP1 and UCP4 are more distant from UCP1 but share a significant homology. On the contrary, other mitochondrial carrier proteins such as adenine nucleotide translocator proteins (ANT) displayed lower homology and are evolutionnary more distant. The inventors decided to study the five mitochondrial carrier proteins more closely related to UCP1 namely UCP1, 2, 3, 4 and BMCP1 (Figure 1A). Using semi quantitative RT-PCR, they studied the expression pattern of UCPs in tissues primarily affected in ALS (lumbar spinal cord and skeletal muscle) of wild type animals and extended those studies to liver, brown adipose tissue (BAT) and white adipose tissues (WAT) as control tissues. As expected, UCP1 is primarily expressed in BAT and hardly detectable in the other tissues tested. UCP2 is mainly expressed in WAT but is also highly expressed in the spinal cord. UCP3 expression is high in the skeletal muscle and very low in BAT, WAT and spinal cord. UCP4 and BMCP1 were only hardly detectable in the spinal cord (Figure 1B).

### UCPs pattern of expression in ALS mice tissues

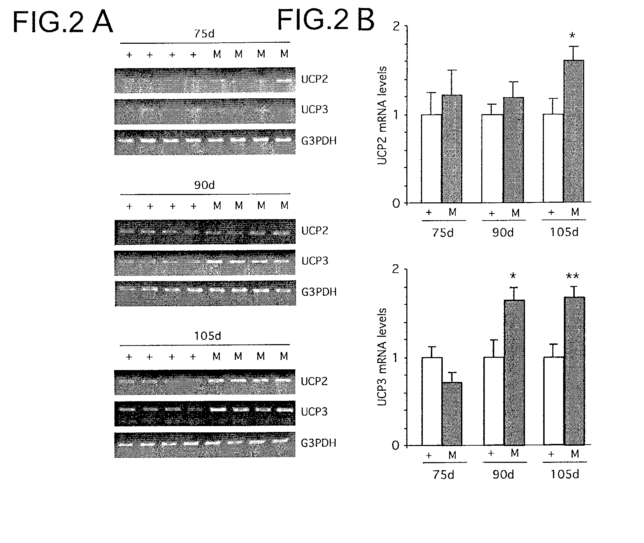

The inventors next studied the expression levels of UCPs in tissues of ALS affected mice carrying a mutated form of SOD1 (G86R mice) using semi quantitative RT-PCR. They focused on three tissues : liver regarding its key importance in energy homeostasis, spinal cord and skeletal muscle because they are primary targets of ALS disease. The inventors measured UCP expression levels at three different ages (75, 90 and 105 days of age) in both G86R mice and control littermates. At 75 and 90 days of age, G86R mice are healthy, with no obvious locomotor symptom. End stage disease sets up at about 105 days of age. They assessed the expression levels of the 5 UCPs along with G3PDH as a control in the liver and found that neither UCP1, 3, 4 nor BMCP1 were significantly expressed in the liver of G86R or control littermates animals. As expected, UCP2 expression was detectable in the liver but its expression levels were unchanged in G86R animals at the three ages tested (data not shown). UCP2, 3, 4 and BMCP1 were expressed at various levels in both wild type and G86R lumbar spinal cords but their expression did not change throughout the disease (data not shown). On the contrary, in skeletal muscle, the inventors found that UCP2 expression was heavily increased at 105 days of age while UCP3 mRNA was upregulated at 90 and 105 days of age (Figure 2). In this tissue, UCP1 and BMCP1 expression were not detectable while UCP4 mRNA levels were low and did not show any variation throughout the disease (data not shown).

### ATP levels are selectively depleted in skeletal muscle tissue of ALS mice

In order to determine whether the detected UCP2 and 3 upregulations in skeletal muscle tissue of ALS mice were able to lead to a functional uncoupling, the inventors measured ATP levels in gastrocnemius muscle, liver and spinal cord of ALS mice. Measured ATP levels in 105 days old mice gastrocnemic muscles were 28,8 +/- 2,2 µmol ATP/µg of proteins in wild type mice and 17,9+/-2,0 µmol ATP/µg of proteins in G86R muscle tissues (n=4 ; p<0,05 as determined using student's t test). ATP levels were found unchanged in both liver and spinal cord tissue (data not shown). A similar ATP depletion was observed in muscles of 90-days old G86R mice (29,4+/-2,5 µmol ATP/µg of proteins vs 20,7+/-1,5 µmol ATP/µg of proteins of proteins, n=4, p<0,05 as determined using student's t test) and in muscles of 75-days old G86R mice (28,9+/-2,6 µmol ATP/µg of proteins vs 16,1+/-3,5 µmol ATP/µg of proteins of proteins, n=3, p<0,05 as determined using student's t test). These results suggest that UCP2 and 3 overexpression in skeletal muscle tissue of ALS mice leads to functional uncoupling and ATP depletion.

### UCP2 but not UCP3- upregulation is linked to denervation processes

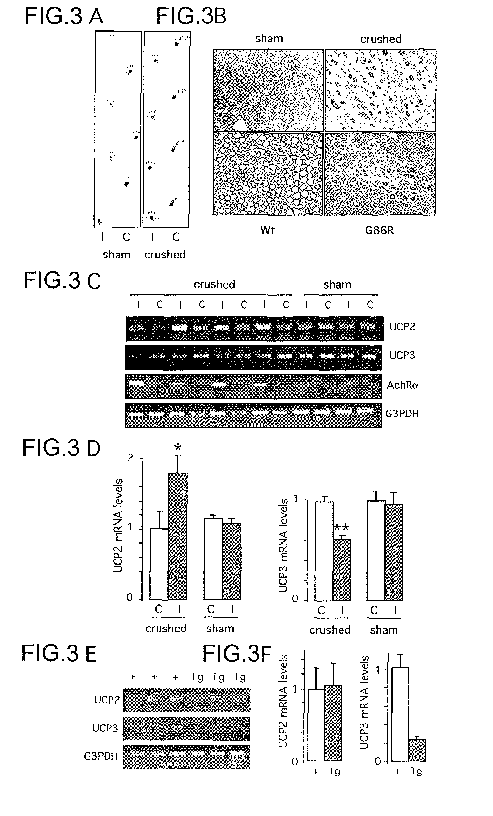

The inventors and others have previously observed that characteristic features of denervation processes occur in G86R animals before the onset of locomotor symptoms. The inventors asked whether UCP2 and UCP3 changes in expression in G86R muscles were part of the typical pattern of expression occurring in response to denervation. As expected, sciatic nerve crush or axotomy provoked hind limb disabilities (Fig. 3A), axonal degeneration (Fig. 3B), and high levels of AchRα mRNA (Fig. 3C) indicative of denervation. RT-PCR analysis revealed a significant up-regulation of UCP2 mRNA both in sciatic nerve crushed and in axotomized (data not shown) animals thus reproducing the situation observed in G86R animals (Fig. 3C and 3D). This was not the case for UCP3 expression which was downregulated in nerve crushed and axotomized animals, a situation opposite to the one observed in G86R transgenic mice. UCP3 upregulation in G86R mice is therefore not linked to denervation processes.

### UCP3 pattern of expression in ALS mice is specific to the mutation of SOD1 rather than to overexpression of SOD1

To determine whether the pattern of UCP2 and UCP3 expression in muscle of G86R animals is due to the overexpression of a superoxide dismutase rather than to expression of a mutated form of this enzyme, semi-quantitative RT-PCR analyses were performed in C57B1/6xDBA/2 mice overexpressing the human wild-type SOD1. No variations in muscular UCP2 expression were noted while a sharp downregulation of UCP3 mRNA was observed (figure 3E and 3F). These data strongly suggest that the early up-regulation of UCP3 mRNA in the skeletal muscle of G86R animals is specifically linked to the expression of the ALS-associated mutant enzyme.

### Muscular UCP3 upregulation is associated with human ALS

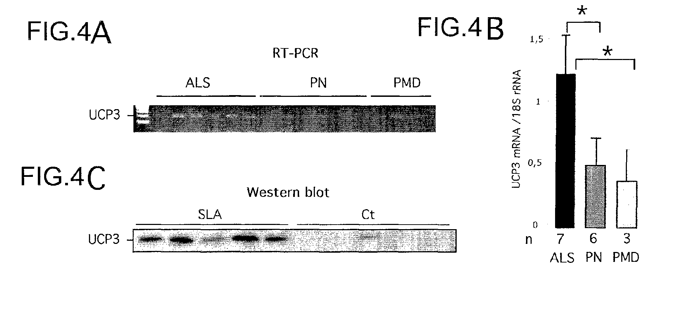

The inventors next assessed the expression levels of UCP3 in human ALS biopsies. As shown on figure 4A, total levels of UCP3 mRNA were increased in the 7 ALS biopsies tested as compared to control patients affected either by neurogenic denervation diseases (polyneuropathies, n=6) or primary muscle diseases (n=3). Furthermore, UCP3 protein levels were massively increased in 14 tested ALS biopsies as compared with normal subjects as demonstrated by western blotting on figure 4B. Collectively, these data show a massive dysregulation of UCP3 in the skeletal muscle of sporadic ALS.

### DISCUSSION

The inventors demonstrated that UCP3 mRNA and protein levels are selectively increased in ALS skeletal muscle, both in an animal model and in human biopsies. This modification was selective to muscle since UCP levels were unchanged in the other tissues tested, especially the spinal cord, the main target of the disease. ALS features in skeletal muscle include typical denervation processes. The inventors thus hypothesized that UCP3 upregulation might be part of the transcriptional program in response to denervation. This was not the case since UCP3 levels were downregulated in skeletal muscles of axotomized mice. While they can not formally exclude the possiblity that UCP3 upregulation is secondary to a special form of chronic denervation accompanying ALS, it seems that an event occuring specifically in ALS muscle. This assumption was further supported by the observation that UCP3 mRNA and proteins were systematically increased in ALS patients and neither in normal controls nor in patients with denervation of different origins. On the contrary, muscle UCP2 upregulation is likely to be linked to denervation processes since it occured in G86R mice close to onset of symptoms and was also observed in axotomized mice and in patients with non-ALS denervation. The inventors' results suggest that UCP2 is trancriptionally regulated in response to intracellular events, such as a decrease in electrical/mechanical activity.

It is the first report demonstrating changes of UCPs expression in a neurodegenerative disease. The inventors' findings raise the question of the functional significance of UCP3 upregulation in ALS muscles. Both UCP2 and UCP3 are able to trigger mitochondrial uncoupling in vitro and in vivo as shown by the decreased mitochondrial proton leak of isolated mitochondria from UCP3 -/- animals (Gong et al, 2000; Vidal-Puig et al, 2000) and by the lowering of mitochondrial proton leak in intact thymocytes of UCP2 -/- animals (Krauss et al, 2002). However, even if these proteins are able to significantly uncouple mitochondria, their absence has no effect on whole animal metabolic responses: UCP3 and UCP2 knockout animals are neither obese nor show abnormal responses to fasting or cold-induced thermogenesis (Gong et al, 2000; Vidal-Puig et al, 2000; Arsenijevic et al, 2000; Zhang et al, 2001). It was recently shown that UCP3 is not involved in basal proton conductance in muscular mitochondria but rather in oxidative stress-inducible proton conductance (Echtay et al, 2002a and 2002b). In physiological situations where oxidative stress occurs, such as exercise or fasting, UCP2 and UCP3 uncoupling activities are activated by superoxide anion thus limitating ROS production by mitochondrial respiratory chain. This in turn decreases superoxide levels and UCP uncoupling activity by a feedback loop. Higher levels of ROS are observed in muscle of UCP 3 -/- mice (Vidal-Puig et al, 2000) and in macrophage of UCP 2 -/- mice (Arsenijevic et al, 2000). Furthermore, *in vitro* and *in vivo* experiments show that UCPs mRNA and protein levels are regulated by the level of oxidative stress. Indeed, exposure to oxidative stress up-regulates UCP2 in clonal beta-cells (Li et al, 2001) and in primary cultures of normal rat hepatocytes (Yang et al, 2000). Expression of the UCP2 gene in beta-cells is decreased after exposure with the antioxidants vitamine E and selenite. UCP3 mRNA and protein levels rise in rat muscles after a 24h starvation (Cadenas et al, 1999). Conversely, levels of UCPs are downregulated when oxidative stress is reduced : Expression of the UCP2 gene in beta-cells is decreased after exposure with the antioxidants Vitamine E and Selenite and our own data demonstrate a downregulation of UCP3 in muscles of mice overexpressing the wild type SOD1. In the context of ALS, it can be assumed that upregulation of UCP-3 in muscle occurs as a response to high levels of ROS. Oxidative stress is a general condition in ALS and is not restricted to the nervous system : oxidative stress biomarkers have been detected in the plasma, increase over time and correlate with the severity of the disease (Bogdanov et al, 2000; Bonnefont-Rousselot et al, 2000). Respiratory chain defects and abnormalities of mitochondrial DNA are present in muscle of ALS patients and have been related to oxidative stress damage (Vielhaber et al, 2000). The susceptibility of muscles may be partly related to specific physiologic properties of muscle fibers. Indeed, muscles are intrinsically already sensitive to oxidative stress: muscle accounts for an important fraction of the total oxygen consumption at rest and this fraction is even higher when skeletal muscles switches to maximal activity. Furthermore, superoxide anions are produced at high levels during transition from anaerobiosis (i.e. anaerobic exercise) to reoxygenation (i.e. post exercise) (Du et al, 1998; Bejma and Ji, 1999).

Although the inventors' data showing increased UCPs levels does not formally prove mitochondrial uncoupling, its association with decreased levels of ATP nevertheless favours this interpretation. Indirect evidences exists of uncoupling *in vivo* both in ALS mice and ALS patients : mitochondrial swelling, an event associated with uncoupling (Hunter et al, 1976; Wieckowski and Wojtczak, 1998) is one of the hallmarks of ALS mice mitochondria (Wong et al, 1995; Kong and Xu, 1998; Jaarsma et al, 2001). Furthermore, when mutated SOD1 is stably transfected in a cell line, it induces a decrease in mitochondrial membrane potential (Carri et al, 1997) a finding suggestive of mitochondrial uncoupling. A recent study by Mattiazzi et al (2002) failed to observe uncoupling on isolated mitochondria from ALS mice. However this does not demonstrate that mitochondria are coupled in ALS mice *in vivo*. Indeed, Mattiazzi *et al.* analyzed mitochondria isolated from liver and spinal cord, tissues in which there are no changes in UCPs expression and ATP levels but did not study skeletal muscle mitochondria. It is further highly probable that the short-lived effectors that control mitochondrial uncoupling, especially superoxide anions (Echtay et al, 2002a and 2002b), are lost during mitochondrial isolation.

Although UCP3 upregulation appears as a protective response against oxidative-stress, the experiments of the inventors suggest that *in vivo* muscular mitochondrial uncoupling can also be deleterious for skeletal muscle cells. Indeed, chronic DNP injections, a well known mitochondrial uncoupler, is able to induce pathophysiological hallmarks reminiscent with ALS. A recent report by Couplan and collaborators is consistent with these findings : these authors generated a transgenic line overexpressing UCP1 in the skeletal muscle. Skeletal muscle mitochondria of these mice are chronically uncoupled, a situation reminiscent of our DNP experiments. These authors show that UCP1 overexpression lowers dramatically muscle mass and induces a shift from fast glycolytic fibers towards slow oxidative fibers (Couplan et al, 2002). Muscle mass decrease and fast-to-slow shift in fiber type composition are typical features of denervated muscles (Brunetti et al, 1997; Huey and Bodine, 1998; Windisch et al, 1998). These data are thus in good agreement with the results of inventors' DNP experiments. Another possible deleterious effect of a sharp UCP3 upregulation is its potential effect on general metabolism. Although UCP3 levels have no effect on the basal metabolism in normal conditions, transgenic mice that overexpress UCP3 in muscles are lean and hyperphagic due to hypermetabolism through mitochondrial uncoupling (Clapham et al, 2000). In ALS, weight loss is a common symptom and a clinical study showed that ALS patients are hypermetabolic (Desport et al, 2001). Further studies are needed to determine if upregulation of UCP3 in muscle of ALS patients plays a role in weight loss and hypermetabolism through mitochondrial uncoupling.

As a conclusion, this study demonstrates that UCP3 is abnormally upregulated in skeletal muscles of both ALS patients and ALS mice. Increased expresion of UCP3 appears as a protective response against oxidative stress through uncoupling of muscle mitochondrias but could also induce deleterious muscle and metabolic effects.

### REFERENCES

Arsenijevic, D., et al. (2000) Disruption of the uncoupling protein-2 gene in mice reveals a role in immunity and reactive oxygen species production. *Nat Genet* 26, 435-439.
Bejma, J., and Ji, L. L. (1999) Aging and acute exercise enhance free radical generation in rat skeletal muscle. *J Appl Physiol* 87, 465-470.
Bogdanov, M., et al. (2000) Increased oxidative damage to DNA in ALS patients. *Free Radic Biol Med* 29, 652-658
Bonnefont-Rousselot, D., et al. (2000) Blood oxidative stress in amyotrophic lateral sclerosis. *J Neurol Sci* 178, 57-62
Brooks, B. R. (1994) E1 Escorial World Federation of Neurology criteria for the diagnosis of amyotrophic lateral sclerosis. Subcommittee on Motor Neuron Diseases/Amyotrophic Lateral Sclerosis of the World Federation of Neurology Research Group on Neuromuscular Diseases and the El Escorial "Clinical limits of amyotrophic lateral sclerosis" workshop contributors. *J Neurol Sci* 124 Suppl, 96-107.
Brunetti, O., et al. (1997) Partial transformation from fast to slow muscle fibers induced by deafferentation of capsaicin-sensitive muscle afferents. *Muscle Nerve* 20, 1404-1413.
Cadenas, S., et al. (1999) UCP2 and UCP3 rise in starved rat skeletal muscle but mitochondrial proton conductance is unchanged. *FEBS Lett* 462, 257-260.
Carri, M. T., et al. (1997) Expression of a Cu,Zn superoxide dismutase typical of familial amyotrophic lateral sclerosis induces mitochondrial alteration and increase of cytosolic Ca2+ concentration in transfected neuroblastoma SH-SY5Y cells. *FEBS Lett* 414, 365-368.
Clapham, J. C., et al. (2000) Mice overexpressing human uncoupling protein-3 in skeletal muscle are hyperphagic and lean. *Nature* 406, 415-418.
Couplan, E., et al. (2002) High level of UCP1 expression in muscle of transgenic mice selectively affects muscles at rest and decreases their IIb fiber content. *J Biol Chem 6,* 6
Desport, J. C., et al. (2001) Factors correlated with hypermetabolism in patients with amyotrophic lateral sclerosis. *Am J Clin Nutr* 74, 328-334.
Du, G., et al. (1998) Generation of superoxide anion by mitochondria and impairment of their functions during anoxia and reoxygenation in vitro. *Free Radic Biol Med* 25, 1066-1074.
Duclert, A., et al. (1990) Induction of acetylcholine receptor alpha-subunit gene expression in chicken myotubes by blocking electrical activity requires ongoing protein synthesis. *Proc Natl Acad Sci U S A* 87, 1391-1395.
Dupuis, L., et al. (2002a) Nogo provides a molecular marker for diagnosis of Amyotrophic Lateral Sclerosis. *Neurobiol Dis* 10, 358-365
Dupuis, L., et al. (2002b) denervation is not the primary cause of prion protein downregulation occuring in the spinal cord of a transgenic model of Amyotrophic Lateral Sclerosis. *Ann NY Acad Sci* 973:116-9
Echtay, et al. (2002a) Superoxide activates mitochondrial uncoupling protein 2 from the matrix side: Studies using targeted antioxidants. *J Biol Chem* 7, 7
Echtay, K. S., et al. (2002b) Superoxide activates mitochondrial uncoupling proteins. *Nature* 415, 96-99.
Gong, D. W., et al. (2000) Lack of obesity and normal response to fasting and thyroid hormone in mice lacking uncoupling protein-3. *J Biol Chem* 275, 16251-16257.
Huey, K. A., and Bodine, S. C. (1998) Changes in myosin mRNA and protein expression in denervated rat soleus and tibialis anterior. *Eur J Biochem* 256, 45-50.
Hunter, D. R., et al. (1976) Relationship between configuration, function, and permeability in calcium-treated mitochondria. *J Biol Chem* 251, 5069-5077.
Jaarsma, D., et al. (2001) CuZn superoxide dismutase (SOD1) accumulates in vacuolated mitochondria in transgenic mice expressing amyotrophic lateral sclerosis-linked SOD1 mutations. *Acta Neuropathol (Berl)* 102, 293-305.
Kong, J., and Xu, Z. (1998) Massive mitochondrial degeneration in motor neurons triggers the onset of amyotrophic lateral sclerosis in mice expressing a mutant SOD1. *J Neurosci* 18, 3241-3250.
Krauss, S., et al. (2002) A significant portion of mitochondrial proton leak in intact thymocytes depends on expression of UCP2. *Proc Natl Acad Sci U S A* 99, 118-122.
Li, L. X., et al. (2001) Uncoupling protein-2 participates in cellular defense against oxidative stress in clonal beta-cells. *Biochem Biophys Res Commun* 282, 273-277.
Mattiazzi, M., et al. (2002) Mutated human SOD1 causes dysfunction of oxidative phosphorylation in mitochondria of transgenic mice. *J Biol Chem* 277, 29626-29633.
Ripps, M. E., et al. (1995) Transgenic mice expressing an altered murine superoxide dismutase gene provide an animal model of amyotrophic lateral sclerosis. *Proc Natl Acad Sci U S A* 92, 689-693.
Salvioli S, et al. (1997) JC-1, but not DiOC6(3) or rhodamine 123, is a reliable fluorescent probe to assess delta psi changes in intact cells: implications for studies on mitochondrial functionality during apoptosis. *FEBS Lett* 411, 77-82
Sambrook, J., et al. (1989) Molecular cloning, a laboratory manual. 2nd edition
Smiley ST, et al. (1991) Intracellular heterogeneity in mitochondrial membrane potentials revealed by a J-aggregate-forming lipophilic cation JC-1. *Proc Natl Acad Sci U S A* 88, 3671-5
Vidal-Puig, A. J., et al. (2000) Energy metabolism in uncoupling protein 3 gene knockout mice. *J Biol Chem* 275, 16258-16266.
Vielhaber, S., et al. (2000) Mitochondrial DNA abnormalities in skeletal muscle of patients with sporadic amyotrophic lateral sclerosis. *Brain* 123, 1339-1348.
Wieckowski, M. R., and Wojtczak, L. (1998) Fatty acid-induced uncoupling of oxidative phosphorylation is partly due to opening of the mitochondrial permeability transition pore. *FEBS Lett* 423, 339-342.
Windisch, A., et al. (1998) Fast to slow transformation of denervated and electrically stimulated rat muscle. *J Physiol* 510, 623-632.
Wong, P. C., et al. (1995) An adverse property of a familial ALS-linked SOD1 mutation causes motor neuron disease characterized by vacuolar degeneration of mitochondria. *Neuron* 14, 1105-1116.
Yang, S., et al. (2000) Mitochondrial adaptations to obesity-related oxidant stress. *Arch Biochem Biophys* 378, 259-268.
Zhang, C. Y., et al. (2001) Uncoupling protein-2 negatively regulates insulin secretion and is a major link between obesity, beta cell dysfunction, and type 2 diabetes. *Cell* 105, 745-755.

## Claims

1. A method of detecting the presence of or predisposition to a disease involving a neuromuscular affection in a subject, the method comprising (i) providing a sample from the subject and (ii) detecting the presence of an elevated level of UCP2 and/or UCP3 expression in said sample.

2. A method of assessing the evolution of a disease involving neuromuscular affection in a patient, the method comprising (i) providing a sample from the subject and (ii) determining the level of UCP2 and/or UCP3 expression in said sample.

3. A method of determining the efficacy of a treatment of a disease involving neuromuscular affection in a patient, the method comprising (i) providing a sample from the subject and (ii) determining the level of UCP2 and/or UCP3 expression in said sample.

4. The method according to claim 2, wherein the method comprises an additional step of comparing the expression level to a reference expression level in a sample from said subject at an earlier time point.

5. The method according to claim 3, wherein the method comprises an additional step of comparing the expression level to a reference expression level in a sample from said subject prior to or at an earlier stage of the treatment.

6. The method according to any one of claims 1-5, wherein said detection is accomplished by detecting levels of UCP2 and/or UCP3 transcript.

7. The method according to any one of claims 1-5, wherein said detection is accomplished by detecting levels of UCP2 and/or UCP3 polypeptide.

8. The method according to any one of claims 6-7, wherein said detection is accomplished by detecting a level of UCP2 expression.

9. The method according to any one of claims 6-7, wherein said detection is accomplished by detecting a level of UCP3 expression.

10. The method according to any one of claims 6-7, wherein said detection is accomplished by detecting a level of UCP2 and UCP3 expression.

11. The method according to any one of claims 1-10, wherein said sample is skeletal muscle sample.

12. The method according to any one of claims 1-11, wherein said disease involving a neuromuscular affection is amyotrophic lateral sclerosis.

13. A diagnostic kit for diagnosing the occurrence of a disease involving a neuromuscular affection or assessing a patient's susceptibility to a disease involving a neuromuscular affection, for assessing the evolution of a disease involving a neuromuscular affection in a patient, or for determining the efficacy of a treatment of a disease involving a neuromuscular affection in a patient, comprising products and reagents for determining in a sample from a subject the level of UCP2 and/or UCP3 expression.

14. A method of selecting biologically active compounds against a disease involving neuromuscular affection comprising the steps of : (a) administering a test compound to a non-human subject; (b) determining the level of UCP2 and/or UCP3 expression in a subject sample; (c) comparing the expression level of UCP2 and/or UCP3 to a reference expression level of UCP2 and/or UCP3 in a sample from said subject prior to the administration of the test compound or at an earlier stage after the test compound administration.

15. The method according to claim 15, wherein the determination of the level of UCP2 or UCP3 expression is carried out in skeletal muscle sample.

16. A method of screening and selecting a compound inhibiting the activity of UCP2 and/or UCP3 comprising the steps of : (a) contacting a test compound with a cell expressing UCP2 and/or UCP3; (b) determining the expression level and/or activity of UCP2 and/or UCP3.

17. A pharmaceutical composition comprising a compound inhibiting the activity of UCP2 and/or UCP3.

18. The pharmaceutical composition according to claim 17, wherein said compound is selecting from the group consisting of an antisense or an RNAi against UCP2 and/or UCP3 transcript, and an antibody directed against UCP2 and/or UCP3.

19. Use of a compound inhibiting the activity of UCP2 and/or UCP3 for the preparation of a drug for treating a disease involving neuromuscular affection.

20. Use according claim 20, wherein said compound is selecting from the group consisting of an antisense or an RNAi against UCP2 and/or UCP3 transcript, and an antibody directed against UCP2 and/or UCP3.

21. Use according claim 20 or 21, wherein said disease is amyotrophic lateral sclerosis.
